# EUROPÄISCHE PATENTSCHRIFT

(11) **EP 1 558 596 B1**
(45) Veröffentlichungstag und Bekanntmachung des Hinweises auf die Patenterteilung: **25.06.2008**
(21) Anmeldenummer: 03769439.5
(22) Anmeldetag: 23.10.2003
(51) Int. Cl.: C07D 301/12

(54) **RÜCKFÜHRUNG DES BEI DER OXIDATION VON OLEFINEN NICHT UMGESETZTEN OLEFINS**
RETURNING AN OLEFIN WHICH IS NOT REACTED DURING THE OXIDATION OF OLEFINS
RECYCLAGE DE L'OLEFINE N'AYANT PAS REAGI LORS DE L'OXYDATION D'OLEFINES

(30) Priorität: 23.10.2002 DE 10249378
(43) Veröffentlichungstag der Anmeldung: 03.08.2005
(73) Patentinhaber: BASF SE, 67056 Ludwigshafen (DE)
(72) Erfinder: BASSLER, Peter, 68519 Viernheim (DE); GÖBBEL, Hans-Georg, 67169 Kallstadt (DE); TELES, Joaquim, Henrique, 67166 Otterstadt (DE); RUDOLF, Peter, 68526 Ladenburg (DE)
(74) Vertreter: Huhn, Michael
(86) Internationale Anmeldenummer: PCT/EP2003/011736
(87) Internationale Veröffentlichungsnummer: WO 2004/037802

(56) Entgegenhaltungen:
- EP-A- 0 467 538
- EP-A- 0 646 558
- EP-A- 0 719 768
- WO-A-02/14298
- DE-A- 10 155 470

## Beschreibung

Die Erfindung betrifft ein Verfahren zur kontinuierlichen Rückführung des bei der Epoxidation von Olefinen mit Hydroperoxiden zu Oxiranen nicht umgesetzten Olefins. Dabei wird der bei der Epoxidation entstehende Abgasstrom unter Kühlung verdichtet und in einer Druckdestillation in einen Olefin-haltigen Sumpfstrom und einen Abgasstrom, der weitgehend frei von Kohlenwasserstoffen ist, aufgetrennt. Das Olefin wird in den Epoxidationsprozess zurückgeführt. Besonders vorteilhaft lässt sich das Verfahren zur Rückführung des bei der Herstellung von Propenoxid eingesetzten Propens anwenden. Die Erfindung betrifft auch eine Vorrichtung, mit der das Verfahren durchgeführt werden kann.

Bekanntlich nimmt bei der Epoxidation von Olefinen mit Hydroperoxiden zu Oxiranen mit steigendem Olefinumsatz die Selektivität der Oxiranbildung zugunsten unerwünschter Nebenreaktionen deutlich ab. Um trotzdem eine hohe Selektivität von über 95 % erreichen zu können, werden deshalb insbesondere im technischen Maßstab diese Reaktionen vorzugsweise nur bis zu einem Olefinumsatz von ca. 85 bis 95 % betrieben.

Bekannt ist auch, das nicht umgesetzte Olefin aus dem Reaktionsprozess zu isolieren und dann wieder in den Oxidationsprozess (Epoxidation) zurückzuführen.

So wurde ein Verfahren vorgeschlagen, bei dem ein Gasgemisch, bestehend aus dem Olefin und Sauerstoff, der aus der Zersetzungsreaktion des bei der Epoxidation als Hydroperoxid verwendeten Wasserstoffperoxids stammt, abgetrennt, und das Olefin in einem flüssigen Absorptionsmittel aus dem Gasgemisch absorbiert wird. Dabei muss zum Sauerstoff ein Inertgas in ausreichender Menge zugesetzt werden, um die Bildung entflammbarer Gaszusammensetzungen zu verhindern. In einer bevorzugten Ausführung wird dieses Verfahren zur Wiedergewinnung von Propen aus der Umsetzung von Propen mit Wasserstoffperoxid zu Propenoxid verwendet. Als Inertgas wird vorzugsweise Methan und als flüssiges Absorptionsmittel ein Gemisch, das Isopropanol und Wasser umfasst, eingesetzt (EP-B 0 719 768).

Bei diesem Verfahren wirkt sich die geringe Löslichkeit des Olefins im wasserhaltigen Isopropanol ungünstig aus. Deshalb müssen relativ große Mengen an Lösungsmittel eingesetzt werden, um das Olefin trotzdem durch Absorption aus dem Abgasstrom gewinnen zu können. Ferner ist es unwirtschaftlich, der zur Absorption verwendeten Kolonne zusätzlich zum Abgasstrom ein weiteres Gas, insbesondere Methan, zuzuführen.

Es war daher die Aufgabe gestellt, ein verbessertes Verfahren zur Rückgewinnung des bei der Epoxidation von Olefinen zu Oxiranen verwendeten Olefins bereitzustellen, mit dem eine effektivere Rückgewinnung des Olefins aus dem Abgasstrom als nach dem Verfahren des Standes der Technik möglich ist.

Diese Aufgabe konnte erfindungsgemäß dadurch gelöst werden, dass man den bei der Epoxidation eines Olefins mit Hydroperoxid zum Oxiran entstehenden Abgasstrom unter Kühlung verdichtet, das darin enthaltene Olefin durch Destillation aus dem Abgasstrom isoliert und in den Epoxidationsprozess zurückführt.

Gegenstand der Erfindung ist somit ein Verfahren zur kontinuierlichen Rückführung des bei der Epoxidation von Olefinen mit Hydroperoxid zu Oxiranen nicht umgesetzten Olefins, das im während der Epoxidation entstehenden Abgasstrom enthalten ist, dadurch gekennzeichnet, dass es die Stufen (i) bis (iii) umfasst
(i) Verdichten und Abkühlen des Abgasstromes,
(ii) Abtrennung des Olefins aus dem in Stufe (i) erhaltenen Abgasstrom durch Destillation,
(iii) Epoxidation des in Stufe (ii) abgetrennten Olefins mit Hydroperoxid.

Beim erfindungsgemäßen Verfahren ist es nicht notwendig, in der Stufe der Abtrennung des Olefins zusätzlich ein Inertgas in die Abtrennvorrichtung einzuspeisen, um explosionsfähige Gemische zu vermeiden. Die Isolierung des Olefins über eine Absorptionsanlage entfällt. Hingegen erlaubt es das erfindungsgemäße Verfahren, mit relativ geringem Aufwand bei nur einmaliger Destillation das Olefin bereits in hoher Ausbeute aus dem Abgasstrom abzutrennen. Da das Verfahren kontinuierlich betrieben werden kann, ist es für die technische Anwendung außerordentlich vorteilhaft einsetzbar.

Nach dem Stand der Technik kann die Epoxidation von Olefinen mit Hydroperoxid einstufig oder mehrstufig durchgeführt werden. Solche Methoden, wie auch ein technisches Verfahren, werden beispielsweise in der WO 00/07965 beschrieben.

Zur Abtrennung der bei der Oxidation entstehenden Oxirane können beispielsweise Destillationskolonnen verwendet werden. Hierbei werden Abgasströme am Kopf der Kolonnen erhalten. Diese Abgasströme enthalten als Komponenten stets nicht umgesetztes Olefin und eine geringe Menge Sauerstoff, der aus der Zersetzungsreaktion des eingesetzten Hydroperoxides stammt. Zur besseren Regelung der Destillation werden üblicherweise Inertgase, vorzugsweise Stickstoff, verwendet. Da diese gleichfalls über den Kopf der Kolonnen entnommen werden, enthalten die Abgasströme somit auch diese Gase. Damit ist es beim erfindungsgemäßen Verfahren nicht mehr notwendig, ein zusätzliches Gas zur Vermeidung explosionsfähiger Gemische in die Absorptionsanlage einzuspeisen.

Im Allgemeinen ist es ausreichend, in Stufe (i) den Abgasstrom auf eine Temperatur von vorzugsweise 0 bis 70 °C, mehr bevorzugt 15 bis 55 °C, insbesondere 30 bis 40 °C abzukühlen.

Diese Temperaturbereiche können beispielsweise bereits mit Wasser als Kühlmittel eingestellt werden.

Zur Verdichtung können die üblichen Apparaturen wie beispielsweise Hubkolben-, Membran-, Schrauben- oder Rotationsverdichter eingesetzt werden.

Vorzugsweise erfolgt die Verdichtung nicht in einer Stufe, sondern mehrstufig mit Abkühlung zwischen den einzelnen Verdichtungsstufen. Diese Vorgehensweise hat den Vorteil einer gut zu kontrollierenden Verdichtungsendtemperatur auf den zulässigen Wertebereich.

In einer bevorzugten Ausführung des Verfahrens wird die Verdichtung des Abgasstromes in zwei Stufen durchgeführt.

Nach dem Verdichten steht der Abgasstrom dann vorzugsweise unter einem Druck von 2 bis 30 bar, mehr bevorzugt 10 bis 25 bar, insbesondere 12 bis 20 bar.
Demzufolge ist eine Ausführung des erfindungsgemäßen Verfahrens dann auch dadurch gekennzeichnet, dass in Stufe (i) der Abgasstrom auf eine Temperatur von 0 bis 70 °C abgekühlt und auf einen Druck von 2 bis 30 bar verdichtet wird.

Der durch Abkühlung und Verdichtung vorbehandelte Abgasstrom wird nun einer Destillationskolonne zugeführt, wobei die üblichen Kolonnen verwendet werden können. Solche Kolonnen sind beispielsweise Rektifizierkolonnen mit vorzugsweise zwischen 3 und 20, bevorzugt 5 und 10 theoretischen Trennstufen. Sie können beispielsweise als Packungskolonne mit Füllkörpern oder geordneten Packungen, als Bodenkolonnen oder als Rotationskolonnen ausgeführt werden.

Der verdichtete Abgasstrom wird kontinuierlich in die Kolonne eingespeist. Die Einspeisestelle kann dabei im mittleren Säulenbereich der Kolonne liegen. In Abhängigkeit von den physikalischen Eigenschaften des zu trennenden Stoffgemisches werden nun Druck und Temperatur so gewählt, etwa in den Bereichen wie vorstehend beschrieben, dass es zum teilweisen Verdampfen des zu trennenden Stoffgemisches kommt. Dabei können das Olefin als hochsiedende Komponente des Abgasstroms am Boden der Kolonne und die niedrigsiedenden Komponenten, wie beispielsweise die im Abgasstrom enthaltenen Inertgase oder weitere flüchtige, bei der Reaktion entstandene Nebenprodukte, über den Kopf der Kolonne abdestilliert werden.

Durch den Anteil an inerten Gasen im Abgasstrom verläuft die Stofftrennung außerhalb des Bereiches, in welchem sich explosionsfähige Gemische mit Sauerstoff bilden können. Deshalb erübrigt sich in der Destillationsstufe (ii) eine zusätzliche Einspeisung weiterer Gase, um die Bildung explosionsfähiger Gemische zu verhindern.

Der Gasanteil des Abgasstromes, der mit den Leichtsiedern erhalten wird, kann beispielsweise einer Verbrennung zugeführt werden.

Das im Sumpf der Kolonne erhaltene Olefin fällt vorzugsweise in einer Reinheit von mindestens 90 % an. Es kann im Allgemeinen ohne weitere Reinigungsschritte dem Epoxidationsprozess mit dem Hydroperoxid zugeführt werden.

Beim erfindungsgemäßen Verfahren ist es besonders vorteilhaft, dass das Olefin in dem Maße, wie es aus dem Abgasstrom der Epoxidation abgetrennt wird, wieder in den Epoxidationsprozess zurückgeführt werden kann. Somit ist ein kontinuierlich zu betreibendes Verfahren möglich, das außerordentlich wirtschaftlich ist.

Besonders vorteilhaft kann das erfindungsgemäße Verfahren für die Abtrennung von Propen aus einem Abgasstrom, der aus der Oxidation von Propen zu Propenoxid stammt, durchgeführt werden.

Wie bereits vorstehend ausgeführt, umfasst der Abgasstrom dann neben dem Propen auch Inertgase, insbesondere Stickstoff, und eine geringe Menge Sauerstoff.

Für die Epoxidation von Propen zu Propenoxid ist es nicht notwendig, das Propen in hochreiner Form einzusetzen, sondern es kann in der Qualitätsstufe "chemical grade" verwendet werden. Solches Propen enthält Propan, wobei Propen und Propan etwa im Volumenverhältnis 97 : 3 bis 95 : 5 vorliegen.

In einer besonders bevorzugten Ausführung des erfindungsgemäßen Verfahrens umfasst daher der Abgasstrom als Olefin Propen aus der Epoxidation von Propen zu Propenoxid und Propan.

In dieser Ausführungform des erfindungsgemäßen Verfahrens wird der Abgasstrom bevorzugt auf eine Temperatur von 30 °C bis 40 °C abgekühlt und auf einen Druck von 12 bis 20 bar verdichtet. Ganz besonders bevorzugt sind eine Temperatur von ca. 35 °C und ein Druck von ca. 16 bar.

Besagter verdichteter Abgasstrom wird nun zur Abtrennung in Stufe (ii) in eine Destillationskolonne eingespeist, was vorzugsweise nahezu auf der obersten Trennstufe erfolgt. Demzufolge wird die Kolonne fast als reine Abtriebskolonne betrieben. Dies hat zur Folge, dass innerhalb der Kolonne das verdampfende Gasgemisch nur relativ gering mit den hochsiedenden Komponenten angereichert wird.

Deshalb kommt es zu einer raschen und guten Abtrennung der kohlenwasserstoffhaltigen Fraktion, enthaltend Propen und Propan, als Sumpfprodukt, während die im Abgasstrom enthaltenen Inertgase oder weitere flüchtige, bei der Epoxidation entstandene Nebenprodukte, über den Kopf der Kolonne als Leichtsiederfraktion abdestillieren. Es lassen sich mindestens 93% der Kohlenwasserstoffe aus dem Abgasstrom abtrennen. Die Leichtsiederfraktion weist somit nur noch geringe Konzentrationen an Kohlenwasserstoffen auf, die vorzugsweise unter 7 Gew.-% liegen, wobei die Gesamtsumme der in der Leichtsiederfraktion enthaltenen Komponenten 100 Gew.-% beträgt.

Der über den Kolonnenboden anfallende Strom der Komponenten Propen und Propan kann anschließend in einem C₃-Splitter, wie er beispielsweise beschrieben ist in Ullmann's Encyclopedia of Industrial Chemistry, Vol A22, Seite 214, in die Komponenten Propen und Propan getrennt werden. Die Trennung kann dabei in einer Kolonne bei einem Druck von ca. 15 bis 25 bar erfolgen. Man kann zur Trennung aber auch den C₃-Splitter in Form thermisch gekoppelter Kolonnen ausführen. Diese können dann beispielsweise bei einem Druck von ca. 15 bzw. 25 bar betrieben werden. Das Propen wird dabei über Kopf, das Propan mit dem Sumpf erhalten.

Das Propen kann nun wieder dem Epoxidationsprozess mit dem Hydroperoxid zugeführt werden. Propan kann als Energiequelle zur Dampferzeugung verwendet werden.

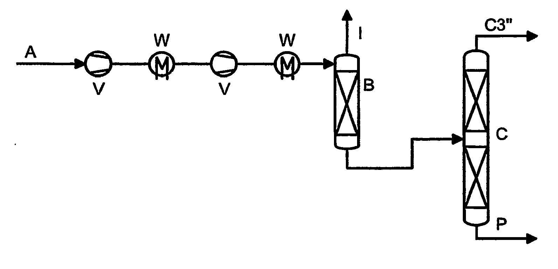

Als Beispiele für Olefine, die nach dem erfindungsgemäßen Verfahren aus Abgasströmen abgetrennt werden können, die bei der Epoxidation besagter Olefine mit Hydroperoxid zu den entsprechenden Oxiranen entstehen, seien folgende Verbindungen genannt:
Ethen, Propen, 1-Buten, 2-Buten, Isobuten, Butadien, Pentene, Piperylen, Hexene, Hexadiene, Heptene, Octene, Düsobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen, Norbornen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z.B. Acrylsäure, Methacrylsäure, Crotonsäure, Maleinsäure, Vinylessigsäure, ungesättigte Fettsäuren, wie z.B. Ölsäure, Linolsäure, Palmitinsäure, natürlich vorkommende Fette und Öle.
Bevorzugt werden in das erfindungsgemäße Verfahren solche Olefine eingesetzt, die unter Normalbedingungen gasförmig vorliegen oder einen Siedepunkt unterhalb 150 °C aufweisen. Vorzugsweise sind dies Olefine mit 2 bis 8 Kohlenstoffatomen. Besonders bevorzugt werden Ethen, Propen und Buten eingesetzt. Besonders bevorzugt wird Propen eingesetzt.
Als Hydroperoxid können für die Epoxidation sämtliche aus dem Stand der Technik bekannten Hydroperoxide, die für die Umsetzung des Olefins geeignet sind, eingesetzt werden. Beispiele für solche Hydroperoxide sind etwa tert.-Butylhydroperoxid oder Ethylbenzolhydroperoxid. Als Hydroperoxid kann auch Wasserstoffperoxid eingesetzt werden, beispielsweise als wässerige Lösung.
Die Abgasströme können dabei auch aus Oxidationsprozessen stammen, bei denen die Umsetzung des Olefins mit dem Hydroperoxid katalysiert wird, beispielsweise durch heterogene Katalysatoren.
In einer besonders bevorzugten Ausführung des erfindungsgemäßen Verfahrens wird Propen aus einem Abgasstrom abgetrennt, der bei der Epoxidation von Propen mit Wasserstoffperoxid zu Propenoxid erhalten wird, wobei die Reaktion heterogen katalysiert wird.
Die Erfindung betrifft auch eine Vorrichtung zur Durchführung des erfindungsgemäßen Verfahrens, die dadurch gekennzeichnet ist, dass sie wenigstens einen Reaktor zur Herstellung von Propenoxid, wenigstens eine Apparatur zur Verdichtung des Abgasstroms, wenigstens eine Destillationskolonne zur Abtrennung besagten Propens und Propans aus dem Abgasstrom und einen C₃-Splitter zur Trennung von Propen und Propan umfasst.
Das in der Figur dargestellte Fließbild verdeutlicht, wie nach dem erfindungsgemäßen Verfahren Propen aus der Epoxidierung (Oxidation) von Propen zu Propenoxid zurückgewonnen werden kann, wobei die Verdichtung in zwei Stufen mit dazwischen liegender Abkühlung erfolgt
Bezugszeichenliste für die Figur
   - A: Abgas aus Epoxidierung
   - V: Verdichter
   - W: Wärmetauscher
   - I: Inertgase (N₂), O₂
   - B: Destillationskolonne
   - C: C3-Splitter
   - P: Propan und eventuell weitere Hochsieder
   - C3": Propen "chemical grade"

## Patentansprüche

1. Verfahren zur kontinuierlichen Rückführung eines bei der Epoxidation von Olefinen mit Hydroperoxid zu Oxiranen nicht umgesetzten Olefins, das im während der Epoxidation entstehenden Abgasstrom enthalten ist, **dadurch gekennzeichnet, dass** es die Stufen (i) bis (iii) umfasst
(i) Verdichten und Abkühlen des Abgasstromes,
(ii) Abtrennung des Olefins aus dem in Stufe (i) erhaltenen Abgasstrom durch Destillation,
(iii) Epoxidation des in Stufe (ii) abgetrennten Olefins mit Hydroperoxid.

2. Verfahren nach Anspruch 1, **dadurch gekennzeichnet, dass** in Stufe (i) der Abgasstrom auf einen Druck von 2 bis 30 bar verdichtet und auf eine Temperatur von 0 bis 70 °C abgekühlt wird.

3. Verfahren nach Anspruch 1 oder 2, **dadurch gekennzeichnet, dass** die Verdichtung in Stufe (i) in mindestens zwei Stufen erfolgt.

4. Verfahren nach einem der Ansprüche 1 bis 3, **dadurch gekennzeichnet, dass** der Abgasstrom Propen und Propan umfasst.

5. Verfahren nach Anspruch 4, **dadurch gekennzeichnet, dass** in Stufe (i) der Abgasstrom auf eine Temperatur von 30 bis 40 °C abgekühlt und auf einen Druck von 12 bis 20 bar verdichtet wird.

6. Verfahren nach Anspruch 5, **dadurch gekennzeichnet, dass** das nach der Destillation in Stufe (ii) im Sumpf der Kolonne enthaltene Gemisch aus Propen und Propan in einem C₃-Splitter in Propen und Propan getrennt wird.

7. Vorrichtung zur Durchführung eines Verfahrens gemäß Anspruch 6, **dadurch gekennzeichnet, dass** die Vorrichtung wenigstens einen Reaktor zur Herstellung von Propenoxid, wenigstens eine Apparatur zur Verdichtung des Abgasstroms, wenigstens eine Destillationskolonne zur Abtrennung von Propen und Propan aus dem Abgasstrom und einen C₃-Splitter zur Trennung von Propen und Propan umfasst.

## Claims

1. A process for the continuous recirculation of an olefin which has not been reacted in the epoxidation of olefins by means of hydroperoxide to give oxiranes and is present in the offgas stream formed during the epoxidation, which comprises the steps (i) to (iii)
(i) compressing and cooling the offgas stream,
(ii) separating the olefin from the offgas stream obtained in step (i) by distillation,
(iii) epoxidizing the olefin separated off in step (ii) by means of hydroperoxide.

2. The process according to claim 1, wherein, in step (i), the offgas stream is compressed to a pressure of from 2 to 30 bar and cooled to from 0 to 70°C.

3. The process according to claim 1 or 2, wherein compression occurs in at least two stages in step (i) .

4. The process according to any of claims 1 to 3, wherein the offgas stream comprises propene and propane.

5. The process according to claim 4, wherein, in step (i), the offgas stream is cooled to from 30 to 40°C and compressed to a pressure of from 12 to 20 bar.

6. The process according to claim 5, wherein the mixture of propene and propane present in the bottoms from the column after the distillation in step (ii) is separated into propene and propane in a C₃ splitter.

7. An apparatus for carrying out a process as defined in claim 6, which comprises at least one reactor for preparing propene oxide, at least one apparatus for compressing the offgas stream, at least one distillation column for separating propene and propane from the offgas stream and a C₃ splitter for separating propene and propane.

## Revendications

1. Procédé en vue du recyclage en continu d'une oléfine n'ayant pas réagi lors de l'époxydation d'oléfines à l'aide d'hydroperoxyde pour former des oxirannes, qui est contenue dans le flux de gaz d'échappement se formant lors de l'époxydation, **caractérisé en ce qu'**il comprend les étapes (i) à (iii)
(i) Condensation et refroidissement du flux de gaz d'échappement,
(ii) Séparation par distillation de l'oléfine hors du flux de gaz d'échappement obtenu à l'étape (i),
(iii) Époxydation de l'oléfine séparée à l'étape (ii) à l'aide d'hydroperoxyde.

2. Procédé selon la revendication 1, **caractérisé en ce que**, à l'étape (i), le flux de gaz d'échappement est condensé à une pression de 2 à 30 bars et est refroidi à une température de 0 à 70 °C.

3. Procédé selon la revendication 1 ou 2, **caractérisé en ce que** la condensation à l'étape (i) se fait en au moins deux étapes.

4. Procédé selon l'une quelconque des revendications 1 à 3, **caractérisé en ce que** le flux de gaz d'échappement comprend du propène et du propane.

5. Procédé selon la revendication 4, **caractérisé en ce que** à l'étape (i) le flux de gaz d'échappement est refroidi à une température de 30 à 40 °C et est condensé à une pression de 12 à 20 bars.

6. Procédé selon la revendication 5, **caractérisé en ce que** le mélange de propène et de propane, obtenu après la distillation à l'étape (ii) dans le puits de la colonne, est séparé en propène et en propane dans un dispositif de séparation C₃.

7. Dispositif en vue de l'exécution d'un procédé selon la revendication 6, **caractérisé en ce que** le dispositif comprend au moins un réacteur en vue de la fabrication d'oxyde de propène, au moins un appareil en vue de la condensation du flux de gaz d'échappement, au moins une colonne de distillation en vue de la séparation du propène et du propane hors du flux de gaz d'échappement et un dispositif de séparation C₃ en vue de la séparation du propène et du propane.
